# EUROPÄISCHE PATENTANMELDUNG

(11) **EP 0 673 631 A1**
(43) Veröffentlichungstag der Anmeldung: **27.09.1995**
(21) Anmeldenummer: 94116295.0
(22) Anmeldetag: 15.10.1994
(51) Int. Cl.: A61F 17/00, A45F 3/04, A45C 9/00

(54) **Rucksack zur Aufnahme von Utensilien für die Notfallmedizin**

(30) Priorität: 24.02.1994 DE 4405951
(71) Anmelder: Heraeus Med GmbH, D-63450 Hanau (DE)
(72) Erfinder: Tietze, Bernd, D-63571 Gelnhausen (DE)
(74) Vertreter: Kühn, Hans-Christian

(57) **Zusammenfassung**

Die Erfindung betrifft einen Rucksack zur Aufnahme von Utensilien für die Notfallmedizin mit einem annähernd rechteckigen Rückwandteil (1) und einem über ein erstes Seitenwandteil (7) mit dem Rückwandteil (1) verbundenen aufklappbaren Vorderwandteil (9), das in seiner Fläche dem Rückwandteil (1) etwa entspricht, und mit einem Trägersystem. Um einen deraratigen Rucksack zu schaffen, der eine Übersicht über seinen Inhalt auf den ersten Blick gewährleistet und bei dem die Utensilien für die Notfallmedizin durch den Benutzer leicht entnommen werden können, ohne daß zuerst andere Bestandteile des Rucksack-Inhalts entfernt werden müssen, ist an dem Rückwandteil (1) dem ersten Seitenwandteil (7) gegenüberliegend ein zweites Seitenwandteil (7) angeordnet, welches mit einem aufklappbaren Zwischenwandteil (8) verbunden ist. Es sind weiterhin an den freien Seiten des Rückwandteils (1) zwei Endteile (5) einander gegenüberliegend angeordnet, die bei geschlossenem Rucksack die Seitenwandteile (7) und das Vorderwandteile (9) mindestens teilweise umgreifen und es weisen wenigstens das Rückwandteil (1), das Zwischenwandteil (8) und das Vorderwandteil (9) Mittel zur Befestigung oder Aufnahme der Utensilien für die Notfallmedizin auf.

## Beschreibung

Die Erfindung betrifft einen Rucksack zur Aufnahme von Utensilien für die Notfallmedizin mit einem annähernd rechteckigen Rückwandteil und einem über ein erstes Seitenwandteil mit dem Rückwandteil verbundenen aufklappbaren Vorderwandteil, das in seiner Fläche dem Rückwandte etwa entspricht und mit einem Trägersystem.

Ein derartiger Rucksack ist beispielsweise als "Thomas Emergency Medical Pack" bekannt. Dieser Rucksack ist in Form eines Koffers aufgebaut mit einem Unterteil und einem aufklappbaren Deckel. An dem Unterteil sind Trageriemen angeordnet. An Unterteil und Deckel sind Taschen zur Aufnahme von Utensilien fest angeordnet, das Innere des Rucksackes nimmt verschiedene Utensilien für die Notfallmedizin auf, die paßgerecht in dem Rucksack angeordnet sind. Dieser Rucksack garantiert allerdings keine maximale Übersicht über den Inhalt, da die Anordnung der Utensilien teils übereinander erfolgt, teils sind die Ecken und Seiten des Unterteils nicht einsehbar, so daß eine Übersicht auf den ersten Blick nicht gewährleistet ist. Dadurch ist die Handhabung erschwert, weil der Benutzer stets genötigt ist, einen Teil des Rucksack-Inhalts zu entfernen, um Zugang zu anderen Utensilien zu erlangen.

Der Erfindung liegt deshalb die Aufgabe zugrunde, einen Rucksack nach dem Oberbegriff des Anspruchs 1 zu schaffen, der eine Übersicht über seinen Inhalt auf den ersten Blick gewährleistet und bei dem die Utensilien für die Notfallmedizin durch den Benutzer leicht entnommen werden können, ohne daß zuerst andere Bestandteile des Rucksack-Inhalts entfernt werden müssen.

Die Aufgabe wird erfindungsgemäß dadurch gelöst, daß an dem Rückwandteil dem ersten Seitenwandteil gegenüberliegend ein zweites Seitenwandteil angeordnet ist, welches mit einem aufklappbaren Zwischenwandteil verbunden ist, daß an den freien Seiten des Rückwandteils zwei Endteile einander gegenüberliegend angeordnet sind, die bei geschlossenem Rucksack die Seitenwandteile und das Vorderwandteil mindestens teilweise umgreifen und daß wenigstens das Rückwandteil, das Zwischenwandteil und das Vorderwandteil Mittel zur Befestigung oder Aufnahme der Utensilien für die Notfallmedizin aufweisen. Eine derartige Anordnung gewährleistet, daß der Rucksack zu einer einzigen ebenen Fläche aufgeklappt werden kann, auf der die Utensilien für die Notfallmedizin übersichtlich nebeneinander angeordnet sind. Die Aufgliederung dieser ebenen Fläche ermöglicht eine Anordnung der Utensilien in Gruppen entsprechend ihrem speziellen Verwendungszweck, so daß der Benutzer sofort einen Überblick gewinnt und erkennt, in welchem Teil des Rucksackes die gerade benötigten Utensilien angeordnet sind, so daß er sich nur noch auf die von ihm benötigte Utensilien-Gruppe zu konzentrieren braucht und die anderen Teile der Rucksackfläche bzw. der Utensilien unbeachtet lassen kann, ohne sie erst beiseite räumen zu müssen. Dadurch ist ein schnellerer und sicherer Zugriff zu den einzelnen Materialien gewährleistet, die Gefahr einer Verwechslung verschiedener Utensilien ist praktisch ausgeschaltet.

Zweckmäßig ist es, daß auch die Seitenwandteile und/oder die Endteile Mittel zum Befestigen oder zur Aufnahme von Utensilien für die Notfallmedizin aufweisen. Dadurch kann die gesamte zur Verfügung stehende Fläche ausgenutzt werden. Vorteilhafterweise sind die Mittel zum Befestigen als Haftflächen (sogenannte Krepp- und Flauschverschlüsse) oder als Halteschlaufen ausgebildet. Derartige Befestigungsmittel sichern eine einfache und schnelle Entnahme der einzelnen Utensilien. Es ist möglich, die einzelnen Utensilien unterschiedlich zu gruppieren (beispielsweise nach Verwendungszweck) oder einzelne Utensilien gegen andere auszutauschen, so daß an den gleichen Befestigungsmitteln unterschiedliche Utensilien angeordnet werden können. Zweckmäßig ist es, daß die Endteile Taschen aufweisen, um hier zusätzliche Unterbringungsmöglichkeiten zu schaffen, beispielsweise für kleinere Gegenstände, die sich an Haftflächen oder in Halteschlaufen nicht unterbringen lassen.

Vorteilhaft ist es weiterhin, daß die Mittel zur Aufnahme der Utensilien der Notfallmedizin als Taschen ausgebildet sind. Diese Taschen können aus einem durchsichtigen Material gebildet sein und ebenso wie die Taschen der Endteile zur Aufnahme kleinerer oder anderweitig schlecht befestigbarer Utensilien dienen. Es ist auch möglich, daß die Taschen an Haftflächen der Wandflächen abnehmbar angeordnet sind, um ein schnelles Austauschen der Taschen oder eine zweckentsprechende Anordnung zu gewährleisten. Die Taschen des Rucksackes können zweckmäßigerweise standardisiert sein, d. h. Größe und Aufbau sind so gewählt, daß die Taschen auch in andere Transportbehältnisse eingesetzt werden können. Dadurch ist es möglich, mit einer Serie aufeinander abgestimmter Transportbehältnisse unterschiedlichen Einsatzbedingungen in der Notfallmedizin zu genügen. Außerdem sichert diese universelle Einsetzbarkeit der Taschen auch ihre flexible Anordnung innerhalb des Rucksackes, um für unterschiedliche Einsatzfälle die optimale Anordnung zu ermöglichen. Bei einer derartigen standardartigen Ausführung bzw. Ausbildung der Taschen ist auch eine einfachere und kostengünstigere Nachversorgung von Ersatz- oder Verschleißteilen möglich.

Im folgenden wird die Erfindung anhand eines Ausführungsbeispiels mittels einer Zeichnung näher erläutert.

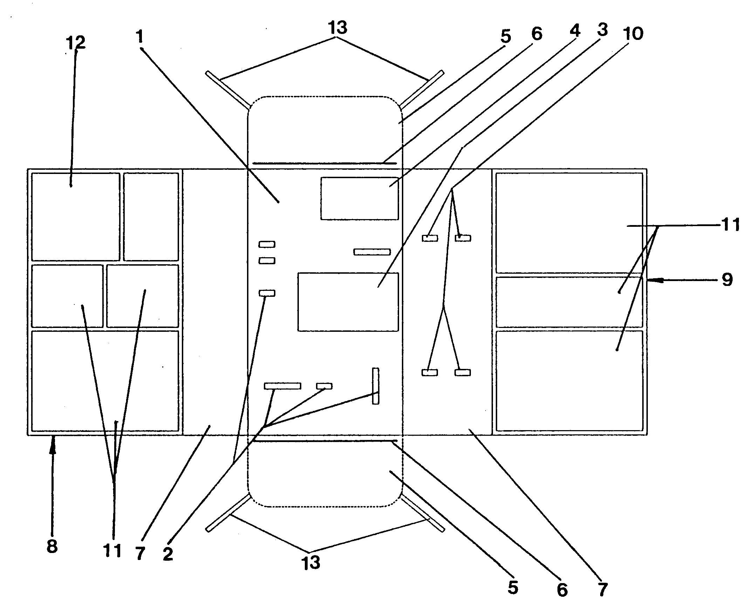

Die Zeichnung zeigt einen erfindungsgemäßen Notfallrucksack, vollständig geöffnet.

Der Rucksack weist als zentrales Teil das Rückwandteil 1 auf, an dessen Rückseite nicht sichtbar ein herkömmliches, vorzugsweise hochwertiges Tragegestell in für Rucksäcke bekannter Art angeordnet ist. An seiner Vorderseite, die dem Rucksackinneren zugewendet ist, weist das Rückwandteil 1 mehrere Haftflächen 2 auf, an denen Taschen oder andere Utensilien der Notfallmedizin befestigt werden können. Desweiteren sind an dem Rückwandteil 1 ein Sauerstoffgerät 3 und eine Absaugpumpe 4 angeordnet. Die Anordnung der Absaugpumpe 4 und des Sauerstoffgerätes 3 enfolgt an dem Rückwandteil 1 deshalb, da diese relativ schweren Geräte dort am sichersten befestigt werden können und den höchsten Tragekomfort gewährleisten. An der Ober- und der Unterseite des Rückwandteiles 1 sind Endteile 5 angeordnet, die mit Reißverschlüssen 6 verschließbare Taschen aufweisen. Links und rechts sind an dem Rückwandteil 1 Seitenwandteile 7 angeordnet, an deren eines sich das Zwischenwandteil 8 und an deren zweites sich das Vorderwandteil 9 anschließt. An dem das Rückwandteil 1 und das Vorderwandteil 9 verbindenden Seitenwandteil 7 sind Halteschlaufen 10 angeordnet, mittels derer längere Gegenstände, wie beispielsweise Schienen in dem Rucksack befestigt werden.

An dem Zwischenwandteil 8 und dem Vorderwandteil 9 sind Taschen 11 mittels in der Zeichnung von den Taschen 11 überdeckter Haftflächen befestigt. Diese Taschen enthalten z. B. Verbandsmaterial, Ausstattungen für Notintubation oder andere Utensilien. Weiterhin ist an dem Zwischenwandteil 8 ein Ampullenetui 12 angeordnet. Ampullenetui 12 und die Taschen 11 sind aus einem transparenten Material gefertigt, so daß der Inhalt sofort erkennbar ist.

Eine derartige Anordnung gestattet eine nahezu beliebige Umgruppierung der Utensilien für die Notfallmedizin innerhalb des Rucksackes, die Taschen können je nach Verwendungszweck bestückt und entsprechend gruppiert werden, so daß der Rucksack beispielsweise innerhalb einer Gruppe verletzter Personen derart ausgebreitet werden kann, daß unmittelbar in der Nähe jeder verletzter Person die entsprechenden Utensilien angeordnet sind. Die Utensilien können auch sehr leicht und verwechslungssicher entnommen und zur Notfallversorgung verwendet werden.

Der Rucksack wird in der Weise geschlossen, daß zuerst das Zwischenwandteil 8 über das Rückwandteil 1 geklappt und danach das Vorderwandteil 9 über das Zwischenwandteil 8 gelegt wird. Anschließend werden die beiden Endteile 5 über die jeweiligen Enden der Wandteile gezogen. Der Verschluß des Rucksackes erfolgt zusätzlich mittels der Verschlußbänder 13.

## Patentansprüche

1. Rucksack zur Aufnahme von Utensilien für die Notfallmedizin mit einem annähernd rechteckigen Rückwandteil (1) und einem über ein erstes Seitenwandteil (7) mit dem Rückwandteil (1) verbundenen aufklappbaren Vorderwandteil (9), das in seiner Fläche dem Rückwanddteil (1) etwa entspricht und mit einem Trägersystem, dadurch gekennzeichnet, daß an dem Rückwandteil (1) dem ersten Seitenwandteil (7) gegenüberliegend ein zweites Seitenwandteil (7) angeordnet ist, welches mit einem aufklappbaren Zwischenwandteil (8) verbunden ist, daß an den freien Seiten des Rückwandteils (1) zwei Endteile (5) einander gegenüberliegend angeordnet sind, die bei geschlossenem Rucksack die Seitenwandteile (7) und das Vorderwandteil (9) mindestens teilweise umgreifen und daß wenigstens das Rückwandteil (1), das Zwischenwandteil (8) und das Vorderwandteil (9) Mittel zur Befestigung oder Aufnahme der Utensilien für die Notfallmedizin aufweisen.

2. Rucksack nach Anspruch 1, dadurch gekennzeichnet, daß die Seitenwandteile (7) und/oder die Endteile (5) Mittel zum Befestigen oder zur Aufnahme von Utensilien für die Notfallmedizin aufweisen.

3. Rucksack nach Anspruch 1 oder 2, dadurch gekennzeichnet, daß die Mittel zum Befestigen als Haftflächen (2) oder als Halteschlaufen (10) ausgebildet sind.

4. Rucksack nach einem der Ansprüche 1 bis 3, dadurch gekennzeichnet, daß die Endteile (5) Taschen aufweisen.

5. Rucksack nach einem der Ansprüche 1 bis 4, dadurch gekennzeichnet, daß die Mittel zur Aufnahme der Utensilien der Notfallmedizin als Taschen (11) ausgebildet sind.

6. Rucksack nach Anspruch 5, dadurch gekennzeichnet, daß die Taschen (11) an Haftflächen (2) der Wandflächen abnehmbar angeordnet sind.
